(19) Europäisches Patentamt
European Patent Office
Office européen des brevets

(11) Publication number: 0 214 721 A1

(12) # EUROPEAN PATENT APPLICATION

(21) Application number: 86305132.2

(51) Int. Cl.4: A61M 25/00 , A61L 29/00

(22) Date of filing: 02.07.86

(30) Priority: 05.07.85 GB 8517091

(43) Date of publication of application: 18.03.87 Bulletin 87/12

(84) Designated Contracting States: CH DE FR GB IT LI NL SE

(71) Applicant: FRANKLIN MEDICAL LIMITED
Cressex Industrial Estate Turnpike Road
High Wycombe Buckinghamshire HP12 3NB(GB)

(72) Inventor: Lipman, Roger David Arnold
6 Grange Road
Chiswick London W4 4AA(GB)

(74) Representative: Raynor, John et al
W.H. Beck, Greener & Co 7 Stone Buildings
Lincoln's Inn
London WC2A 3SZ(GB)

(54) **Balloon catheters.**

(57) A catheter comprising a lumen (2), and being provided with an inflatable balloon (10), wherein the balloon (10) has a laminated construction, and comprises a rubber latex layer (13) with an overlying layer (11) of a polyurethane.

EP 0 214 721 A1

## BALLOON CATHETERS

This invention relates to catheters, that is to say, a tube for use in a body cavity, and in particular to balloon catheters, for example of the type used for drainage of the bladder via the urethra. Such catheters are commonly referred to as Foley catheters. Catheters according to the invention are also useful as catheters for the venous and arterial systems, for example as embolectomy catheters, and catheters used in cardiovascular diagnostics, such as Swan-Ganz catheters. Catheters in accordance with the invention may also be used as naso-gastric tubes and endotracheal tubes.

Foley urinary drainage catheters have an inflatable balloon near the catheter tip to hold the catheter in position in the bladder.

The balloon of the Foley catheter was originally designed simply to retain the catheter in place, and hence a standard indwelling Foley catheter is generally known is a retention catheter. In such a catheter, the balloon is generally approximately 5cc in volume.

Foley catheters are also often used post-operatively, to help stop or control bleeding, by applying pressure to the wound bed. A Foley catheter design for this purpose is known as a hemostatic catheter. Hemostatic catheters generally utilise a balloon of larger volume, for example 30cc. Because of various size and pressure requirements, a hemostatic catheter with a nominal volume of 30cc should be capable of being overinflated to betwen 45cc and 60cc. There are therefore rather stringent requirements for the material of which the balloon is a Foley catheter is formed, particularly if the catheter is to remain in position for an extended period. Such balloons are normally inflated with sterile water, or saline, or, occasionally, dye solutions.

The following are desirable qualities of a catheter balloon.

### Strength

The balloon must be sufficiently strong to withstand normal tension from patient movement whilst in position. A hemostatic catheter must be sufficiently strong to withstand 2-3 lbs of traction for as long as three days in a post-operative patient.

### Symmetry

This refers to the overall roundness of the catheter balloon. A round balloon is important, in that it ensures that the tip is correctly located, and that no occlusion of the eyes occurs. Because the perfectly symmetrical balloon does not exist, a manufacturing tolerance usually sets parameters for defining balloon symmetry.

### Memory

The ability of the catheter balloon to deflate properly, flush to the shaft, is called balloon memory or hysteresis. A balloon with poor memory will remain wrinkled or partially filled, and will cause trauma to the patient upon removal.

There are three materials from which balloon catheters are usually constructed. Latex, PVC, and silicone.

Latex Foley catheters are generally made of several layers of dipped natural rubber latex. They can be used in place in the bladder for up to 10 days. They are commodity items for every day use, and relatively low in cost.

Latex catheters suffer from a number of disadvantages, for example in that chemicals in the latex tends to irritate the sensitive urethra, and urinary salts deposit on the surface, and block the eys and the lumen. Furthermore, the soft nature of latex necessitates thick walls so that only a small lumen is possible, and the lumen can be caused to collapse by suction. Lastly, fluid tends to permeate between the layers of the latex, causing the latex to swell. This results in pain to the patient, and may eventually block off the inflation channel, so that the balloon will not deflate.

Conventional PVC catheters are produced having both a PVC balloon, and a latex balloon. The use of PVC for the main lumen of the catheter has the advantage that it is more rigid, so that thinner walls can be used, giving a larger lumen for better drainage. The material is also less prone to irritation of the sensitive urethra, and less prone to attract urinary salts, which tend to block the eyes and the lumen. Furthermore, PVC is a thermoplastic material, and its relative stiffness when cold assists insertion, whereas after insertion, the catheter softens for patient comfort. PVC catheters can be used continuously for from 4 to 6 weeks. The construction is versatile, and a wide range of different tips is possible. However, the natural rubber latex balloons usually used on such catheters suffer from the same drawbacks as those of latex catheters.

Silicone rubber is a synthetic product with many of the desirable properties of both PVC and latex. In particular, it is very inert to body tissues and fluids, causes a minimum of inflammatory and foreign body reactions, and does not support bacterial growth. It is however expensive, and for this reason, it is not used extensively. A further disadvantage of all-silicone catheters is that the strength of the balloon is relatively poor, and also the lumen size is relatively small.

Catheters have also been produced from silicone coated latex, but these also are not entirely satisfactory, in that they have been found to delaminate under certain circumstances, and cause blockage and trauma.

U.K. patent specification No. 1581955 relates to a method of producing a catheter in which the ends of an inflation cuff are recessed into the catheter shaft, and mentions that composites or laminates of certain material can be utilised. However there is no exemplification or indication of what may be envisaged in the way of composites or laminates, nor any indication that any particularly advantageous properties may be obtained by the use of selected laminate arrangements.

We have now found that an improved catheter can be produced using a specific laminated construction, using a balloon having an outer polyurethane layer and an inner layer of a rubber latex.

In accordance with the invention, there is therefore provided a catheter comprising a lumen, and being provided with an inflatable balloon, wherein the balloon has a laminated construction, and comprises a rubber latex layer with an overlying layer of a polyurethane.

The balloon is formed with the polyurethane layer as the outermost layer, and may conveniently be produced by forming a polyurethane layer on a rubber latex, for example by dipping. In addition to the laminated balloon construction, the catheter in acordance with the invention may also incorporate other portions formed of a latex layer with an overlying poluyurethane layer, in any part of the catheter in which it is desired to combine water-impermeability with flexibility and/or extensibility.

In one embodiment, a catheter may be formed having a main lumen formed of PVC, or, preferably, of polyurethane and the lumen defining a retention balloon or hemostatic balloon formed on the surface thereof from a latex sleeve. A polyurethane coating may be formed over the latex sleeve and distal end of the PVC or polyurethane main lumen by dipping, spraying, or various other methods.

Preferred polyurethanes are block copolymers containing blocks of low molecular weight polyesters or polyethers, linked by a urethane group.

$$-(\underset{\underset{H}{|}}{N}-\underset{\underset{O}{\|}}{C}-O)-$$

Such polyurethanes may be obtained in rigid, semi-rigid, or flexible forms. These materials in generally have excellent biocompatibility, outstanding hydrolytic stability, superior abrasion resistance, excellent physical strength, and high flexure endurance. They are also resistant to gamma radiation, oils, acids, and bases.

Polyurethanes suffer from the difficulty that thin films allow the transport of moisture. Hence, use of an unmodified polyurethane balloon on a catheter is precluded because the water-filled balloon would deflate over time, and the catheter retention means would fail. In the balloon in accordance with the invention, water passage is prevented by the use of the latex layer.

Another disadvantage to the use of polyurethane alone as a material for catheter balloons is its hysteresis, or memory properties, especially compared to natural rubber latex or silicone rubber. In the catheter in accordance with the invention, this disadvantage is overcome by providing a laminated structure, in which the desired flexibility is provided by the latex layer.

The preferred method of producing the laminated portion is by dipping. Thus, a pre-fabricated catheter having a portion, for example the flexible balloon, made of rubber latex may be over-dipped with an aqueous polyurethane emulsion. A suitable polyurethane emulsion is Witco W-290H, manufactured in the U.K. by Baxenden Chemicals, of Accrington, Lancashire.

Alternatively, a polyurethane colloid, such as Witco W-234 may be utilised in the post-dipping step. The colloid is a water-based polyurethane composition, which contains a co-solvent, such as N-methyl pyrrolidone, which aids the adhesion of the polyurethane to the natural rubber latex.

A preferred method is to overcoat the preformed latex balloon with a solution of polyurethane. For this purpose, a polyurethane such as Tecoflex SG80A manufactured by Thermedics Inc., Woburn, Mass., U.S.A. may advantageously be used. This material is an aliphatic polyurethane, with excellent biocompatibility . The solvent may be a polar solvent, for example tetrahydrofuran, methylene chloride, N-methyl pyrrolidone, or N-methyl acetamide, or a blend of two of more of these solvents, or one or more of these solvents with a co-solvent such as ethyl acetate or n-butyl acetate. A tetrahydrofuran solution of SG80A at 10% W/V level, and having a viscosity of about 2200 cps at 25°C is particularly valuable for producing well adhering coatings to natural rubber latex.

It is preferred in the preparation of a balloon catheter that the formation of eyes or drainage holes is carried out after the formation of the polyurethane layer, so the the inside of the catheter lumen does not become filled with the polyurethane composition. A balloon catheter in accordance with the invention may be formed, for example, by coating a catheter as disclosed in U.K. Patent No. 1380991 with a polyurethane composition.

A preferred embodiment of the invention is illustrated in the accompanying drawings, in which:-

Figure 1 is an elevation of a catheter in accordance with the invention,

Figures 2 and 3 respectively show the proximal and distal ends of the catheter on a larger scale, and partially in section,

Figure 4 is a fragmentary section of the distal end of the catheter, and

Figure 5 is a fragmentary sectional view of the distal end of the catheter, showing the balloon inflated.

Referring to the drawings, a tube 1 of a plastics material, for example polyurethane or plasticised polyvinyl chloride, is provided with a funnel assembly 7 at its proximal end, and a balloon 10 at its distal end. The tube 1 has a drainage lumen 2 communicating with a hole 4 formed in its distal end, and an inflation lumen 3, communicating with the interior space of a balloon 10.

A side arm 12 of the funnel 7 communicates with the inflation lumen 3 via an eye 6. The distal and proximal ends of the lumen 3 are plugged by plugging elements 8 and 9 respectively, of extruded cord or wire, which may be of plasticised PVC. Plugging or closing may however be effected in other ways, for example using an X-ray opaque material.

The tip of the catheter may be shaped as shown in Figures 3 to 5, by shaping the end of the extruded tube on a high frequency forming machine.

The funnel 7 may be bound to the proximal end of the tube 1 with appropriate adhesive, for example cylohexanone/tetrahydrofuran a polyurethane adhesive such as Tecoflex adhesive I-PP, or mixtures of two or more thereof.

A vulcanised rubber latex inflation sleeve 13 is positioned on the tube to cover a hole 5 formed in lumen 3. The sleeve 13 is attached to the PVC or polyurethane tube 1 at its ends, by any suitable method, for example as described in U.K. Patent Specification no. 1380991 to define the lumen of the balloon 10. A polyurethane coating 11 is then formed on the outer surface of the distal portion of the catheter, including the balloon 10, by dipping the distal end of the catheter in a solution of polyurethane in an appropriate solvent.

Several catheters in accordance with Figures 1 to 5 above and having a polyurethane tube 1 were produced by dipping the distal ends of catheters into a 10% W/V solution of Tecoflex SG-80A polyurethane in tetrahydrofuran, prepared by dissolving the polymer in the solvent in a rotating drum overnight at room temperature. The drainage hole 4 in the catheter was formed only after the polyurethane coating had been produced. The catheters were dipped to a depth of about 1cm above the balloon, and then withdrawn, using a vertical carriage. The rate of dipping and withdrawal was 40 mm/minute, and the catheters were air dried overnight, and then further dried at 50°C in a forced air oven. Eyes 4 were punched on two opposite sides of the drainage lumen 2.

A number of catheters in accordance with the invention were compared with standard latex Foley catheters, PVC catheters with a latex balloon, and all-silicone catheters, according to the following test.

The catheters were passed through holes in a tank, such that the balloons of the catheters were inside the tank, and the balloons were inflated with sterile saline solution. The inflated balloons thus served to block the holes in the tank, and to prevent leakage of liquid. The tank was filled with simulated urine solution, having a pH of approximately 6.6 The composition of the simulated urine was as described in British Standard Specification No. BS 1695: 1981 -Specification for Sterile Urinary Catheters for Single Use.

The simulated urine drained through the eyes in the catheters above the balloon, and was collected below the funnels in a second tank. The simulated urine was pumped continously from the second tank back up to the upper tank, containing the catheters under test. In this way, continous drainage of urine from catheters could be simulated over a long period of time. In this way, encrustation tendencies could be compared.

The results are shown in Table I.

## TABLE I

## SIMULATED URINE TEST AT 37°C

| CATHETER DESCRIPTION | ELAPSED TIME (DAYS) | OBSERVATION |
|---|---|---|
| All Latex Foley Catheter (size 14 Ch 20 ml balloon) | 66 | Encrusted balloon Eyes blocked Restricted Flow |
| PVC Catheter with latex balloon (size 16 Ch 20 ml balloon) | 121 | Encrusted balloon Eyes starting to block Restricted flow |
| All-Silicone Catheter (size 20 Ch 5 ml balloon) | 152 | Encrustation starting to form on balloon |
| Polyurethane Catheter with polyurethane coated latex balloon (size 14 Ch 20 ml balloon) | 180+ | No visible encrustation |

In accord with convention, catheter sizes (outside diameters) in the Tables are given in Ch (Charriere) units, where 1 Ch = 0.333 mm.

The results show that the all latex catheter was the first to become encrusted with salts, the PVC catheter was next, followed by the all-silicone catheter. The polyurethane catheter with the polyurethane coated natural rubber latex balloon did not become encrusted, even after 180 days on test.

The catheters in accordance with the invention were then tested to destruction, according to the following test method.

The balloon catheter was placed in a conical shaped jig, 1 mm larger in internal diameter than the external diameter of the catheter over the balloon area. The catheter was clamped, inflated to 1½ times the specified capacity with water, and pulled at a constant rate of 20" per minute, and the force required to separate the balloon from the tube was recorded.

The type of failure exhibited was recorded, and tabulated according to the following key.

A -Peeled Funnel End
B -Balloon Burst
C -Shaft Broke
D -Peeled Balloon End
E -Balloon Delaminated

The results are recorded in Table 2.

TABLE 2

| TYPE OF CATHETER CONSTRUCTION | FORCE TO FAILURE, KG | TYPE OF FAILURE |
|---|---|---|
| PVC shaft, polyurethane coated latex balloon (size 12-16 Ch 5 ml balloon) | 19.2, 12.7, 13.0, 18.5 | A,C |
| Polyurethane shaft Polyurethane coated latex balloon (size 12-16 Ch 5 ml balloon) | 15.4, 12.3, 8.2, 11.9 | A,C |
| All-silicone catheter (size 12-16 Ch 5 ml balloon) | 6.0, 6.9, 5.8, 4.5 | B,C |

As can be seen from Table 2, in spite of this rigorous test, in no case did the composite polyurethane-coated latex balloon peel from the shaft, delaminate, or burst. Further, the balloons were substantially stronger than the all-silicone catheters.

In a further test, the balloon of each of the catheters was inflated, and placed in freshly prepared simulated urine at 37°C for 14 days. They were then washed and deflated, and the balloons were deflated. No unacceptable ridges were present on the balloon, which might traumatise the urethra upon catheter removal.

Although the invention has been described with particular reference to balloon catheters for drainage of the bladder, it will be appreciated that the composite latex/polyurethane construction may readily be utilised in other forms of catheter, and various other modifications may be made, within the scope of the appended claims.

The catheters in accordance with the invention may be utilised in various forms of medical treatment.

## Claims

1. A catheter comprising a lumen, and being provided with an inflatable balloon, wherein the balloon has a laminated construction, and comprises a rubber latex layer with an overlying layer of a polyurethane.

2. A catheter as claimed in Claim 2, wherein the laminated construction is produced by forming the said balloon of the rubber latex material, and forming a layer of a polyurethane material on the outer surface thereof.

3. A catheter as claimed in Claim 1 or Claim 2, wherein the laminated construction is formed by a dipping process.

4. A catheter as claimed in any one of claims 1 to 4 having at least two lumens.

5. A catheter as claimed in any one of the preceding claims, which is a Foley urethral catheter, a cardiovascular catheter, an embolectomy catheter, or a thrombectomy catheter.

6. A method of producing a laminated catheter, which method comprises forming a layer of a polyurethane material on at least a portion of the surface of an inflatable latex balloon formed on the catheter.

7. A method as claimed in Claim 8, wherein the layer of polyurethane material is formed by using an aqueous-based polyurethane emulsion.

8. A method as claimed in Claim 8, where in the layer of polyurethane material is formed by using an organic solvent based solution of polyurethane.

9. A method as claimed in any one of Claims 6 to 8, wherein the polyurethane layer is formed by a dipping process.

10. A method as claimed in claim 1, wherein the catheter has main lumen formed of a polyurethane material.

FIG.1.

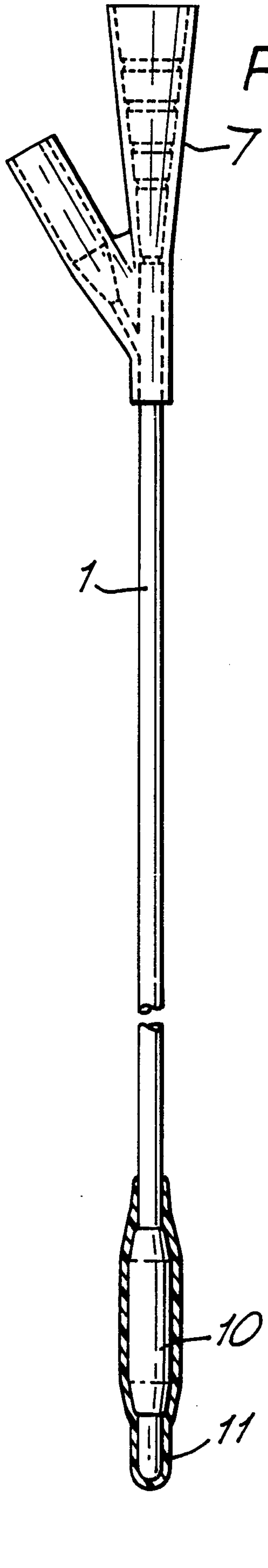


FIG. 2.

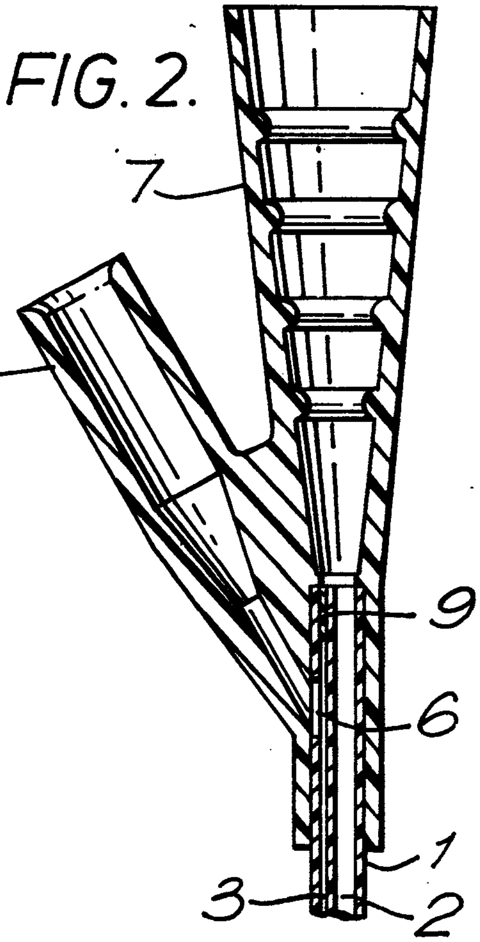


FIG. 4.

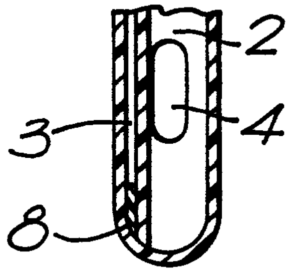


FIG.3.

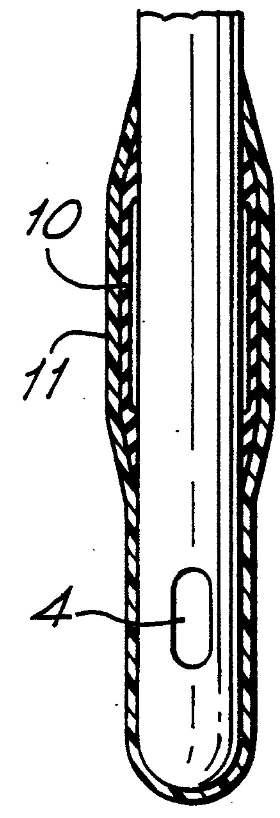


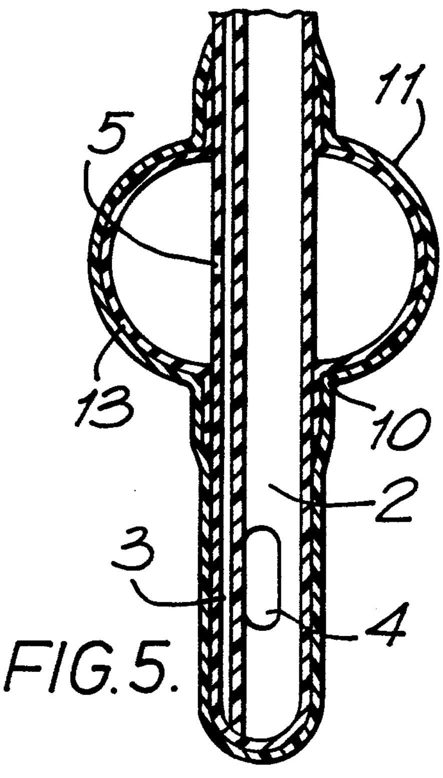


FIG.5.

European Patent Office

# EUROPEAN SEARCH REPORT

Application number

EP 86305132.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DD - A - 152 474 (W.GUENDEL et al.) * Totality; especially page 2, last paragraph; page 3, fourth paragraph * -- | 1-6,8, 9 | A 61 M 25/00 A 61 L 29/00 |
| Y | US - A - 4 100 309 (M.J.MICKLUS et al.) * Totality; especially column 1, line 63 - column 2, line 2; column 5, lines 18-23 * -- | 1-6,8, 9 | |
| A | FR - A - 2 328 482 (SHERWOOD MED.) * Totality; especially page 5, line 23 - page 6, line 11 * -- | 1-6,8-10 | |
| A | US - A - 3 539 674 (P.PERENIUK) * Totality * -- | 1-6,8-10 | TECHNICAL FIELDS SEARCHED (Int. Cl.4) |
| A | US - A - 3 336 918 (N.C.JECKEL) * Totality * -- | 1 | A 61 M 25/00 A 61 M 29/00 A 61 L 29/00 |
| A | GB - A - 1 467 928 (KENDALL COMP.) * Totality * -- | 1 | |
| A | FR - A - 2 188 448 (W.WARNER & COMP.) * Totality * ---- | 1 | |

The present search report has been drawn up for all claims

| Place of search | Date of completion of the search | Examiner |
|---|---|---|
| VIENNA | 18-11-1986 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82